# EUROPEAN PATENT APPLICATION

(11) **EP 4 410 327 A1**
(43) Date of publication of application: **07.08.2024**
(21) Application number: 24155231.4
(22) Date of filing: 01.02.2024
(51) Int. Cl.: A61M 5/14, A61M 5/142, A61M 5/145

(54) **INFUSION SYSTEM WITH SENSOR SYSTEM HAVING REDUCED COMPLIANCE SENSITIVITY**

(30) Priority: 02.02.2023 US 202363483012 P
(71) Applicant: Fresenius Kabi USA, LLC, Lake Zurich, IL 60047 (US)
(72) Inventor: POWERS, Benjamin, Lake Zurich, 60047 (US)
(74) Representative: Maikowski & Ninnemann Patentanwälte Partnerschaft mbB

(57) **Abstract**

An infusion system includes an infusion device, an infusion set, and a sensor assembly. The infusion device includes a syringe including a barrel with an outlet end and a plunger, and a pusher device in engagement with the plunger. The set occludes an infusion line having a first end in communication with the outlet end of the barrel and a second end, and a backpressure regulator disposed between the first end and the second end of the infusion line. The sensor assembly includes a first sensor disposed along the infusion line between the backpressure regulator and the second end of the infusion line. The infusion device also includes a controller coupled to the sensor assembly.

## Description

### TECHNICAL FIELD

The present disclosure is generally directed to infusion systems and methods. More particularly, the present disclosure is directed to infusion systems and methods incorporating sensor systems having reduced sensitivity to the compliance of the components of the infusion system.

### BACKGROUND

One important feature of a modern infusion system is its ability to detect occlusions occurring in an associated infusion line. Occlusions can cause the delivery of a medical fluid, for example a medication or a nutritional fluid, to vary from a prescribed delivery rate. In extreme cases, occlusions can prevent delivery of the medical fluid completely. Consequently, it is important to be able to detect occlusions, and to do so in a timely manner.

The detection of an occlusion may be performed in a number of different ways. Where the infusion system includes a syringe pump where an electromotive drive acts on a plunger of a syringe, the detection of an occlusion may be determined from a signal provided by a force sensor disposed between the plunger (e.g., the plunger head) and the electromotive drive (e.g., a pusher device or mechanism). According to one example, the signal may be compared with a threshold, and an occlusion may be determined if the signal exceeds the threshold.

While such a detection system benefits from its simplicity, it is also subject to the compliance of the components of the syringe pump, and in particular the components of the syringe. Compliance may be referred to as a measurement of the degree to which a component deforms under force. When referencing a syringe pump, one may refer to the compliance of the syringe body (i.e., barrel) as well as the compliance of the plunger. Considering that the above detection system involves a force measured at the plunger head, both the compliance of the syringe body and the plunger must be considered. As a result of the compliance of the syringe (both body and plunger), a sensor system like that described above to be slow, or slower, to detect occlusions, and may vary in accordance with the compliance of the components.

### SUMMARY

According to a first aspect, an infusion system includes an infusion device, an infusion set, and a sensor assembly. The infusion device includes a syringe including a barrel with an outlet end and a plunger, and a pusher device in engagement with the plunger. The set includes an infusion line having a first end in communication with the outlet end of the barrel and a second end, and a backpressure regulator disposed between the first end and the second end of the infusion line. The sensor assembly includes a first sensor disposed along the infusion line between the backpressure regulator and the second end of the infusion line. The infusion device also includes a controller coupled to the sensor assembly.

### BRIEF DESCRIPTION OF THE DRAWINGS

The disclosure will be more fully understood from the following description taken in conjunction with the accompanying drawings. Some of the figures may have been simplified by the omission of selected elements for the purpose of more clearly showing other elements. Such omissions of elements in some figures are not necessarily indicative of the presence or absence of particular elements in any of the exemplary embodiments, except as may be explicitly delineated in the corresponding written description. None of the drawings is necessarily to scale.
Fig. 1 is a schematic view of an embodiment of an infusion system including an infusion device (in the form of a syringe pump), an infusion set with a backpressure regulator, and a sensor assembly.
Fig. 2 is an enlarged, perspective view of an embodiment of an infusion system in accord with the schematic of Fig. 1.
Fig. 3 is an enlarged, cross-sectional view of an embodiment of a backpressure regulator as may be used in the embodiments of Figs. 1, 2, 9, and 10.
Fig. 4 is an enlarged, cross-sectional view of another embodiment of a backpressure regulator as may be used in the embodiments of Figs. 1, 2, 9, and 10.
Fig. 5 is an enlarged, cross-sectional view of an additional embodiment of a backpressure regulator as may be used in the embodiments of Figs. 1, 2, 9, and 10.
Fig. 6 is an enlarged, cross-sectional view of a further embodiment of a backpressure regulator as may be used in the embodiments of Figs. 1, 2, 9, and 10.
Fig. 7 is an enlarged, cross-sectional view of a still further embodiment of a backpressure regulator as may be used in the embodiments of Figs. 1, 2, 9, and 10.
Fig. 8 is an enlarged, cross-sectional view of an additional embodiment of a backpressure regulator as may be used in the embodiments of Figs. 1, 2, 9, and 10.
Fig. 9 is an enlarged, perspective view of another embodiment of an infusion system.
Fig. 10 is a frontal view of a still further embodiment of an infusion system.

### DETAILED DESCRIPTION OF VARIOUS EMBODIMENTS

A detailed description of an infusion devices and methods in accordance with the present disclosure is set forth below. It should be understood that the description below of specific devices and methods is intended to be exemplary, and not exhaustive of all possible variations or applications. Thus, the scope of the disclosure is not intended to be limiting, and should be understood to encompass all variations or embodiments that would occur to persons of ordinary skill.

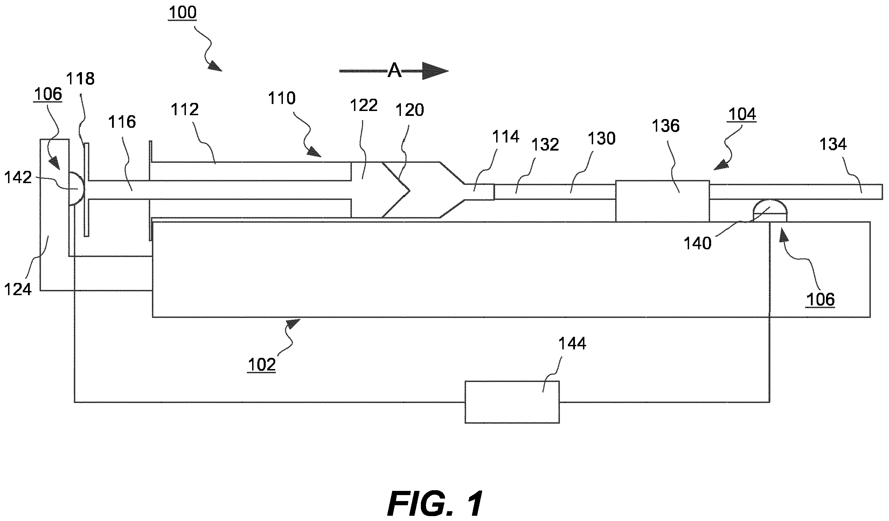

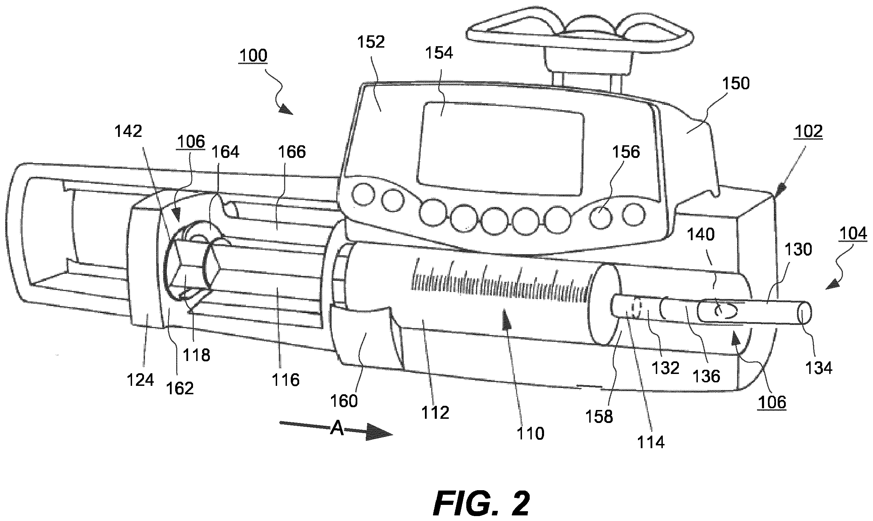

Fig. 1 shows a schematic view of an embodiment of an infusion system, while Fig. 2 shows a perspective view of an embodiment of an infusion system consistent with the schematic of Fig. 1. Consequently, both systems will be referred to as the infusion system 100, and similar components will be indicated with the same reference numerals. The infusion system 100 includes equipment generally grouped into an infusion device, or pump, 102, an infusion set 104, and a sensor assembly 106.

As will be explained below with reference to Fig. 2, the pump 102 may include a housing, and certain aspects of the infusion set and/or the sensor assembly may be connected to or mounted on the housing. Further, the pump 102 may include a controller, and the controller may be coupled to the sensor assembly 106 to receive signals therefrom. Consequently, the description of the system 100 as a pump 102, set 104, and sensor assembly 106 does not require a physical separation of elements, unless specifically stated. Reference to a pump 102, set 104 and sensor assembly 106 is for ease of discussion.

As illustrated in Figs. 1 and 2, the pump 102 includes a syringe 110 with a barrel 112 having an outlet end 114. The syringe 110 also includes a plunger 116 with a first end 118 (or plunger head) disposed outside the barrel 112, and a second end 120 with a piston 122 that is disposed within the barrel 112 (see Fig. 1). The second end 120 and the piston 122 are moveable within the barrel 112 in the direction towards and away from the outlet end 114.

The pump 102 also includes a pusher device 124. As illustrated, the pusher device 124 is in engagement with (e.g., abuts) the first end 118 of the plunger 116. The movement of the pusher device 124 in a first direction causes the piston 122 to be moved along the barrel 112 in the direction of the outlet end 114 (see arrow A, Fig. 1). The movement of the pusher device 124 in a second direction causes the piston 122 to be moved along the barrel 112 in an opposite direction away from the outlet end 114.

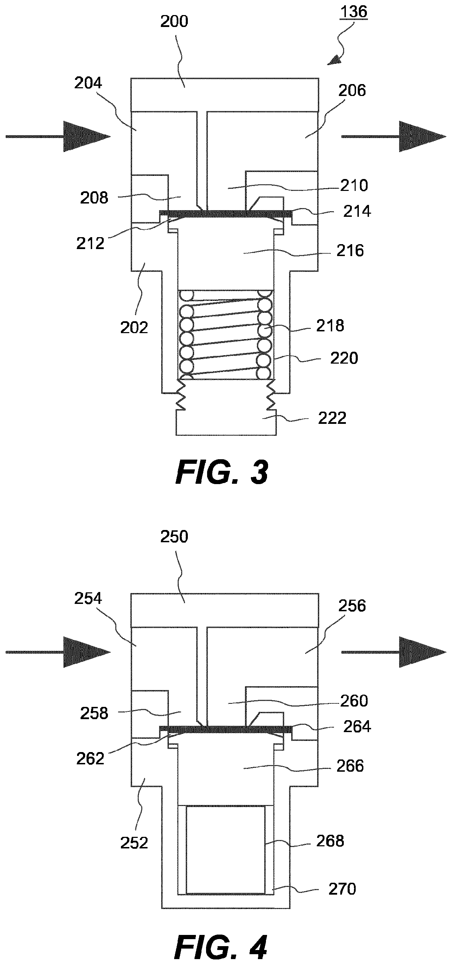

As also illustrated in Figs. 1 and 2, the infusion set 104 includes an infusion line 130 having a first end 132 in communication with the outlet end 114 of the barrel 112. The line 130 also has a second end 134. The second end 134 is connected to a container or to a patient, for example by a catheter connected to the second end 134 (not shown). The line 130 may be made of flexible tubing of a material such as a polyvinylchloride (PVC)I. The set 104 also includes a backpressure regulator 136 disposed between the first and second ends 132, 134 of the line 130, as will be discussed relative to Fig. 3.

As illustrated in Figs. 1 and 2, the sensor assembly 106 includes a first sensor 140 disposed along the infusion line 130 between the backpressure regulator 136 and the second end 134 of the infusion line 130. As is illustrated, the first sensor 140 is disposed outside the infusion line 130, although that need not be the case according to all embodiments. The sensor assembly 106 also includes a second sensor 142 disposed between the first end 118 of the plunger 116 and the pusher device 124.

As illustrated in Fig. 1, the pump 102 also includes a controller 144 coupled (i.e., directly or indirectly connected) to the sensor assembly 106, and thus to the first and second sensors 140, 142. The controller 144 may receive signals from the sensor assembly 106, and use the signals received to control the operation of the pusher device 124. The controller 144 also may receive the signals from the sensor assembly 106, and activate one or more output devices to inform or to alert a user to a condition or state, e.g., a condition of the pump 102 or fluid flowing through the line 130. The controller 144 may include for example a processor and memory (or storage device), electrical circuits, or a combination of a processor and memory and electrical circuits.

For example, in operation, the controller 144 may receive a signal from the first sensor 140 (which may be a force sensor or a pressure sensor), and may use that input to determine if an occlusion has occurred downstream of the regulator 136. For example, the signal from the sensor 140 may be compared to a threshold, and an occlusion may be detected if the signal exceeds the threshold. The determination of such an occlusion condition or state may cause the controller 144 to perform additional actions, such as pausing operation of the pusher device 124 to pause delivery of fluid from the syringe 110 or activating an output device, for example a display or a speaker. In addition, the controller 144 may receive a signal from the second sensor 142 (which also may be a force sensor), and may use that input as part of a control algorithm to deliver fluid from the syringe 110 at a specified fluid flow rate. The fluid may be a medical fluid, for example a medication or a nutritional fluid for the parenteral feeding of a patient.

In any event, it is believed that the inclusion of the backpressure regulator 136 provides one or more advantages when used with such a sensor assembly 106. In particular, the backpressure regulator 136 isolates the pump 102, and in particular the syringe 112, from the line downstream of the regulator 136. As a consequence, the compliance of the syringe (which may include the compliance of the barrel, the pusher, and the line between the barrel and the regulator) can be limited or eliminated from consideration when considering line pressure relative to occlusions downstream of the pump 102. This may result in higher fidelity and quicker occlusion detections. A further advantage is that isolation may limit or eliminate unintended boluses caused when the height of the pump (relative to the container or patient) is changed, or when an occlusion is removed downstream.

Having discussed the system 100 in general terms, further details are discussed relative to Figs. 1-3.

Referring first to Fig. 2, the pump 102 includes a housing 150 having a front face 152 and a display device 154 disposed thereon. The display device 154 may be a touch-sensitive display, allowing a user to enter commands for operation of the pump 102 and displaying operational information regarding the process of an actual infusion operation. The pump 102 may also include a plurality of input devices 156, for example buttons, configured to allow a user to enter commands.

The pump 102 also includes a receptacle 158 in which the syringe 110 is disposed. The pump 102 may also include a holding or fixation device 160, which may include a releasable clamp element, to secure the syringe 110 in position in the receptacle 158. The clamp 160 may also provide a mechanical connection between the pump 102, and in particular the housing 150, and the syringe 1110.

As mentioned above, the pump 102 also includes the pusher device 124 that is in engagement with the syringe 110, and in particular the plunger 116. The pusher device 124 has a face 162 that is in engagement with the first end 118 (or plunger head). The plunger head 118 may be fixed to the pusher device 124 with a holding or fixation device 164, which also may include a releasable clamp. As illustrated, the clamp 164 may be disposed around or about the head 118, such that the head 118 is held in tight abutment with the pusher device 124.

During operation of the pump 102, the pusher device 124 may be electromotively driven in an actuation direction A such that the second end 120 (or piston) is advanced along the barrel 112 (see Fig. 1), and a fluid (such as a medical fluid) contained in the barrel 112 is delivered via the infusion line 130 towards the container or patient. An electromotive drive (e.g., one or more gears connected to an electric motor) may be disposed within the housing 150, and connected to the pusher device 124 by a drive shaft 166. Like the sensor system 106, the electromotive drive also may be coupled to the controller 144, and the controller 144 may be configured (e.g., programmed) to operate the electromotive drive to cause the pusher device 124 to move, e.g., in the actuation direction A, in an opposite direction to the actuation direction A, or to pause (i.e., to move neither in the actuation direction A or in the opposite direction). According to an embodiment, the electromotive drive is configured as described in US Pat. No. 11364338, which is incorporated by reference herein in its entirety.

As mentioned above, the sensor 142 is disposed between the pusher device 124 and the head 118, and may be configured to measure the force exerted on the head 118. Specifically, the sensor 142 may be capable of measuring a force when the pusher device 124 is actuated to push the piston 122 in the actuation direction A into the barrel 112 to deliver a fluid from the barrel 112 in a downstream direction towards the container or patient. Because of the tight abutment between the head 118 and the pusher device 124 (as a consequence of the fixation device 164), the force sensor 142 also may be capable of measuring the force between the pusher device 124 and the head 118 when the pusher device 124 is actuated in a direction opposite to the actuation direction A such that the piston 122 is pulled out of the barrel 112 and a fluid is drawn in an upstream direction through the infusion line 130. According to an embodiment, the force sensor 142 is configured as described in US Pat. No. 9731068, which is incorporated by reference herein in its entirety.

The sensor 140 of the sensor assembly 106 may be mounted on the housing 150 of the pump 102, although in such a way that the sensor 140 will be in contact with the infusion line 130 at a point downstream of the backpressure regulator 136. As mentioned above, the backpressure regulator 136 acts to isolate the compliances of the components of the syringe 110 from the line downstream of the regulator 136. As such, the sensor 140 may be used to determine a pressure in the line 130, which pressure can be used to determine if an occlusion is occurring, without having that pressure influenced by the compliances of the syringe components. As illustrated, the sensor 140, which may be a force sensor, may be disposed so that the line 130 may be disposed abutting the sensor 140.

While not a requirement of the present disclosure, the housing 150 may also be configured to mount the back pressure regulator 136 on the housing 150 as well. According to other embodiments, the regulator 136 may be disposed between the first and second ends 132, 134 of the line 130, but not mounted on or to the housing 150.

As to the regulator 136, Fig. 3 illustrates an embodiment of a backpressure regulator 136 that may be used in the embodiment of the infusion set 104 illustrated in Figs. 1 and 2. The embodiment illustrated in Fig. 3 is merely an example of such a regulator. Other devices capable of providing the necessary isolation of upstream and downstream components may also be included in substitution therefor. For example, several additional embodiments are illustrated in Figs. 4-8, and will be discussed in turn.

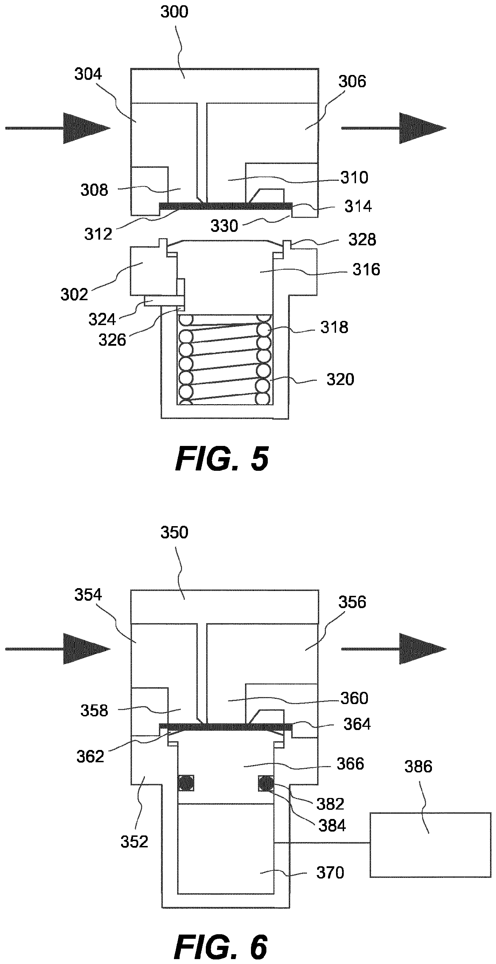

The regulator 136 includes a first housing 200 and a second housing 202. It will be recognized that the first housing 200 and/or the second housing 202 each may be an assembly of multiple individual parts, or a single part (i.e., a unitary part). As illustrated in Fig. 3, the first housing 200 and the second housing 202 may be joined together irreversibly, meaning that the housings 200, 202 cannot be separated without damage to the housings 200, 202. As will be discussed below relative to the embodiment of Fig. 5, the housings 200, 202 may be joined together reversibly through the use of fasteners, for example.

As illustrated, the first housing 200 defines an inlet port 204 and an outlet port 206. The inlet port 204 is in fluid communication with the first end 132 of the infusion line 130. The outlet port 206 is in fluid communication with the second end 134 of the infusion line 130. Fluid flows through the housing 200 between the inlet port 204 to the outlet port 206 when an upstream pressure exceeds a threshold pressure. Fluid is not intended to flow between the outlet port 206 in the direction of the inlet port 204.

The first housing also defines a central outer passage 208 and a central inner passage 210. As illustrated, the central outer passage 208 is annular in shape (at least in part), while the central inner passage 210 is circular in shape. The cross-sectional area of the central inner passage 210 is smaller than the cross-sectional area of the central outer passage 208.

The regulator 136 also includes a sensing diaphragm 212, which has an edge 214 that is disposed between the first and second housings 200, 202 to be held in place therebetween. A first side of the diaphragm 212 is exposed to the fluid passing between the inlet port 204 and the outlet port 206, and in particular between the central outer passage 208 and the central inner passage 210. On a second side of the diaphragm 212 is disposed a piston 216 that abuts the second side of the diaphragm 212, and is biased towards the first housing 200 by a biasing element 218. The biasing element may be, for example, a coil spring, as illustrated in Fig. 3.

As illustrated, the biasing element 218 is disposed in a biasing element chamber 220 formed in the second housing 202. The biasing chamber 220 may be configured (e.g., through the inclusion of a threaded aperture) to accept an adjustment screw 222 therethrough at the end opposite the first housing 200. The adjustment screw acts on the biasing element 218 to set the spring force of the biasing element 218. According to other embodiments, the adjustment screw may not be included (see, e.g., Fig. 4).

The biasing element 218 may be adjustable manually, for example by a user of the infusion system 100 manipulating the adjustment screw 222 to advance or withdraw the screw 222 from the chamber 220 Alternatively, the controller 144 may be coupled to an actuator (e.g., a motor without or without a gear train) that is connected to the screw 222, and the controller 144 may adjust the biasing element automatically, without the need for the user to enter an input for the controller 144 to do so.

In operation, when the fluid acting on the sensing diaphragm in the outer central passage 208 exceeds a threshold pressure (in accordance the opposing forces created by the biasing element 218 and the pressure applied to the diaphragm across the area of the annular region 208), fluid will pass from the inlet port 204, through the outer central passage 208 into the inner central passage 210, and exit the outlet port 206. On the other hand, fluid is prevented from passing from the outlet port 206 to the inlet port 204 by the action of the biasing element 218. More particularly, opposing forces are created by the biasing element 218 (and piston 212) and the pressure applied to the diaphragm across the area of the passage 210. Because the area of the passage 210 is smaller than the area of the passage 208, backflow is limited or prevented unless the downstream pressure (at port 206) is much higher than the pressure required to move fluid from port 204 to port 206.

As a consequence of the backpressure regulator 136, the infusion system 100 is divided into an upstream section and a downstream section. While the pressure of the downstream section remains below the threshold pressure of the regulator, any changes in pressure of the downstream section will not change the pressure in the upstream section. As such, the compliance of the syringe 110 is isolated from compliance on the downstream side of the regulator 136. As such, the readings of the sensor 140 may be relied upon to determine the presence of an occlusion without concern for the compliance of the syringe 100. Further, because downstream pressure changes do not cause changes in pressure in the syringe, the concern for potential unintended boluses caused by changes in pump elevation or a removal of an occlusion may be mitigated. For example, a drop in downstream pressure will not release any fluid volume stored in the compliance of the syringe because the pressure in the syringe will not change.

As mentioned above, Figs. 4-8 illustrate additional embodiments of the backpressure regulator 136. The embodiments may have features in common, and features that are different from those of the other embodiments. In addition, the features of one embodiment may be combined with the features of other embodiments. For example, the embodiment of Fig. 5 illustrates a two-piece regulator that has a biasing element similar to that illustrated in Fig. 3. It will be recognized, however, that the two-piece regulator could be used instead with the biasing elements illustrated in Figs. 4, 6, 7, and 8.

As all of the embodiments of Figs 3-8 have features in common, similar structures are numbered similarly. For example, in the embodiment of Fig. 3, the first and second housing are numbered as 200 and 202. In the embodiment of Fig. 4, the first and second housing are numbered as 250 and 252. Similar remarks can be made about the other embodiments of Figs. 5-8.

Turning first to Fig. 4, the embodiment of the regulator illustrated therein includes a first housing 250 and a second housing 252. As was the case with the embodiment of Fig. 3, the first housing 250 and/or the second housing 252 each may be an assembly of multiple individual parts, or a single part (i.e., a unitary part). The housings may be reversibly or irreversibly joined together, with a diaphragm 262 disposed therebetween (e.g., an edge 264 of the diaphragm 262 disposed between mating surfaces of the housings 250, 252).

As illustrated, the first housing 250 defines an inlet port 254 and an outlet port 256, with the port 254 in fluid communication with the first end 132 of the infusion line 130 and the port 256 is in fluid communication with the second end 134 of the infusion line 130. The first housing also defines a central (annular) outer passage 258 and a central (circular) inner passage 260. A first side of the diaphragm 262 is exposed to the fluid passing between the inlet port 254 and the outlet port 256, and in particular between the central outer passage 258 and the central inner passage 260.

On a second side of the diaphragm 262 is disposed a piston 266 that abuts the second side of the diaphragm 262. The piston 266 is biased towards the first housing 250 by a biasing element 268. As illustrated, the biasing element 268 is disposed in a biasing element chamber 270 formed in the second housing 252.

Unlike the embodiment of the regulator illustrated in Fig. 3, the biasing element 268 according to the embodiment of the regulator illustrated in Fig. 4 is not a spring in the sense of a piece of metal (e.g., length of wire) that is bent to create a spring force that is applied to the piston 266, like in a coil spring. The biasing element 268 may be made of a solid volume of an elastic material, such as a rubber or other elastomer. As one alternative, the biasing element 268 may include a hollow volume filled with a compressible gas or fluid, with an elastomeric wall defining the hollow volume in which the compressible gas or fluid is filled. In any event, the biasing element 268 is disposed in the biasing element chamber 270 to bias the piston 266 in the direction of the diaphragm 262.

The embodiment of the regulator of Fig. 5 illustrates a feature touched on briefly above: a regulator with a first housing 300 and a second housing 302 that are reversibly attached to each other. This is not to suggest that the first housing 300 and/or the second housing 302 may not be an assembly of multiple individual parts irreversibly joined together, or even a single part (i.e., a unitary part). The housings 300, 302 are reversibly attached together to permit the second housing 302, for example, to be more permanently mounted on (or even part of) the reusable housing 150, while the first housing 300 is part of the infusion line 130, which is disposable.

As illustrated, the first housing 300 defines an inlet port 304 and an outlet port 306, with the port 304 in fluid communication with the first end 132 of the infusion line 130 and the port 306 is in fluid communication with the second end 134 of the infusion line 130. The first housing 300 also defines a central (annular) outer passage 308 and a central (circular) inner passage 310. A first side of a diaphragm 312 is exposed to the fluid passing between the inlet port 304 and the outlet port 306, and in particular between the central outer passage 308 and the central inner passage 310.

Unlike the previous embodiments, while the diaphragm 312 is disposed between the first and second housings 300, 302, the diaphragm 312 is irreversibly joined to the first housing 300. This is to simplify the reversible attachment of the first and second housings 300, 302, and because the edge 314 of the diaphragm 312 cannot be held in place between the first and second housings 300, 302 in the same way as described above relative to the embodiments of Figs. 3 and 4. The methods for joining the diaphragm 312 to the first housing 300 may vary according to the materials used for the diaphragm 312 and the first housing 300. For example, where the housing 300 is plastic and the diaphragm 312 is a PVC membrane, the joining methods may include thermal welding and ultrasonic welding.

When the first housing 300 is attached to the second housing 302, the second side of the diaphragm 312 abuts a piston 316. The piston 316 is biased towards the first housing 300 by a biasing element 318. As illustrated, the biasing element 318 is disposed in a biasing element chamber 320 formed in the second housing 302. While Fig. 5 has a biasing element 318 in the form of a coil spring as illustrated, any of the options discussed and illustrated in Fig. 4 may be used instead. As a further alternative, any of the biasing elements illustrated in Figs. 6-8 may be used as well.

It will be recognized that either the piston 316 or the housing 302 may have a stop or other restriction that cooperates with the other of the piston 316 or the housing 302 to prevent the piston 316 from separating from the housing 302 when the first and second housings 300, 302 are not attached. A stop 324 may be inserted through the wall of the biasing element chamber 320 and cooperate with a groove 326 formed in a cylindrical surface of the piston 316, for example. The groove 326 may be sized to permit a certain degree of travel for the piston 316 when the biasing element 318 acts on the piston 316. While a single stop 324 and a single groove 326 have been illustrated, it will be recognized that a plurality of stops and grooves may be used instead.

As mentioned above, the first and second housings 300, 302 are reversibly attached to each other according to this embodiment. To this end, the first and second housings 300, 302 may include mating features that attach the housings 300, 302 together reversibly, permitting separation without damage to one or both of the housings of the regulator, with or without the use of tools. As one example, a rim 328 of the second housing 302 may be received within and be secured within a recess 330 of the first housing 300, either by relying on the friction between the parts 328, 330 or on a positive attachment mechanism, such as a bayonet mount or lock. Other fasteners may be used instead or in addition to such an attachment mechanism.

Referring now to Fig. 6, an embodiment of a regulator is illustrated wherein a piston is biased not by a structure disposed in the biasing element chamber, but the biasing element chamber in combination with a gas or liquid defines a biasing element that biases the piston. As mentioned above, this embodiment may be combined with the embodiment of Fig. 5, and thus the housings of the embodiment of Fig. 6 may be reversibly attached or irreversibly joined together. The embodiment is preferably (but not necessarily) a two-piece system such as is illustrated in Fig. 5, although the stops and grooves are not illustrated in the cross-sectional view of Fig. 6. It will be recognized that the stops and grooves may be visible in a different cross-sectional view, like that illustrated in Fig. 5.

The embodiment of the regulator illustrated in Fig. 6 includes a first housing 350 and a second housing 352. As was the case with the embodiments of Fig. 3-5, the first housing 350 and/or the second housing 352 each may be an assembly of multiple individual parts, or a single part (i.e., a unitary part). The housings 350, 352 may be reversibly or irreversibly joined together, with a diaphragm 362 disposed therebetween (e.g., an edge 364 of the diaphragm 362 disposed between mating surfaces of the housings 350, 352 or attached to the housing 350 as in the embodiment illustrated in Fig. 5).

As illustrated, the first housing 350 defines an inlet port 354 and an outlet port 356, with the port 354 in fluid communication with the first end 132 of the infusion line 130 and the port 356 is in fluid communication with the second end 134 of the infusion line 130. The first housing also defines a central (annular) outer passage 358 and a central (circular) inner passage 360. A first side of the diaphragm 362 is exposed to the fluid passing between the inlet port 354 and the outlet port 356, and in particular between the central outer passage 358 and the central inner passage 360.

On a second side of the diaphragm 362 is disposed a piston 366 that abuts the second side of the diaphragm 362. The piston 366 is biased towards the first housing 350 by a biasing element. Unlike the embodiment of the regulator illustrated in Figs. 3-5, the biasing element is not a separate structure that is disposed in a biasing element chamber 370. Instead, the biasing element chamber 370 is filled with a gas or liquid (more particularly, a compressed gas or liquid), and defines the biasing element.

According to one embodiment, the gas or fluid is introduced into the biasing element chamber 370, and the chamber 370 is closed so that the gas or fluid cannot escape from the chamber. To this end, the chamber 370 may have a closed end, and the piston 366 may have a sealing mechanism, for example a sealing ring or O-ring 382 that limits or prevents the gas or fluid from flowing or escaping around the piston 366. The sealing ring 382 may be fitted in a recess 384 formed in the cylindrical surface of the piston 366, which recess 384 may be an annular recess.

According to another embodiment, as is also illustrated in Fig. 6, a gas or liquid (i.e., fluid) source 386 may be mounted in the housing 150 of the infusion pump 102. For example, the source 386 may be in the form of a pump that is connected to a fluid reservoir. According to another example, the source 386 may be a valve connected to a compressed fluid reservoir, which valve can be controlled (e.g., by the controller 144) to place the chamber 370 in fluid communication with the compressed fluid reservoir. Still other alternatives are possible.

As mentioned above, it may be preferable to use a two-piece assembly for the regulator when a pump, compressed fluid reservoir, or other fluid source is included. This may simplify the connections required between the pump, reservoir or other fluid source and the chamber 370. On the other hand, in an embodiment where the pump, compressed fluid reservoir or the like is not included, but the fluid is sealed in the chamber 370, the entire regulator may be separate from the infusion device 102. Even where the regulator includes a pump or fluid source mounted or mountable on the housing 150, a regulator where the housings 350, 352 are irreversibly joined may be used provided an interface (e.g., a port on the chamber 370) is included on the housing 150 to allow fluid communication between the fluid source 386 and the chamber 370.

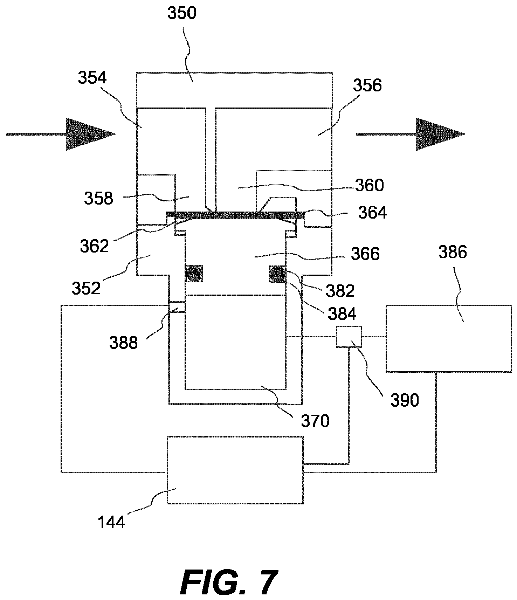

A further embodiment of the regulator is illustrated in Fig. 7. This embodiment is a further modification of the embodiment of Fig. 6, with inclusion of additional equipment. As such, many of the parts of the embodiment of Fig. 7 are numbered as in Fig. 6, and only the additional equipment and functionality of the embodiment of Fig. 7 shall be discussed below.

In addition to the structures described relative to Fig. 6, the embodiment of Fig. 7 includes a sensor 388 and an optional valve 390. The sensor 388 and the valve 390 are coupled to a controller, for example the controller 144, as is the fluid source 386. The controller 144 may be configured (e.g., programmed) to control the fluid source 386 and optional valve 390 to maintain the pressure within the chamber 370 at a particular pressure, or within a particular range of pressures. The pressure within the chamber 370 may be determined by the sensor 388, which generates a signal that is received by the controller 144. The controller 144 may compare the signal to a threshold signal (or an upper and lower threshold signal) to determine if the fluid source 386 and/or valve 390 should be activated to adjust the pressure within the chamber 370. Alternatively, the controller 144 may determine a pressure based on the signal received, and then compare that pressure to a threshold pressure or range of pressures to determine if the fluid source and/or valve 390 should be activated.

Such an embodiment as is illustrated in Fig. 7 permits for control of the pressure in the chamber 370 according to a feedback loop. This may in turn provide for better or more nuanced control of the pressure in the chamber 370.

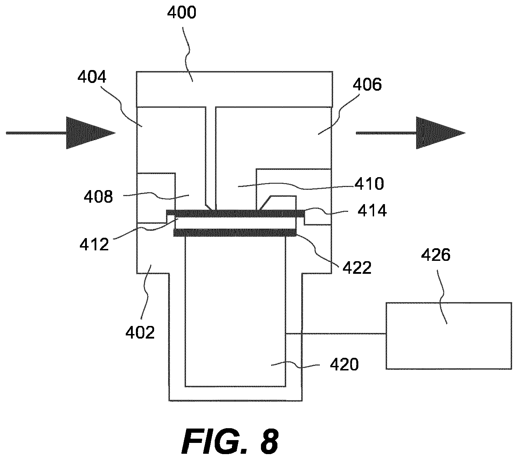

A still further embodiment is illustrated in Fig. 8. Similar to the embodiments of Figs. 6 and 7, this embodiment has a biasing element in the form of a volume of liquid or gas. The embodiment of Fig. 8 lacks the stopper included in the previous embodiments, but may otherwise include much of the same structure and method of operation.

The embodiment of the regulator illustrated in Fig. 8 includes a first housing 400 and a second housing 402. As was the case with the embodiments of Fig. 3-5, the first housing 400 and/or the second housing 402 each may be an assembly of multiple individual parts, or a single part (i.e., a unitary part). The housings 400, 402 may be reversibly or irreversibly joined together, with a diaphragm 412 disposed therebetween (e.g., an edge 414 of the diaphragm 412 disposed between mating surfaces of the housings 400, 402 or attached to the housing 400 as in the embodiment illustrated in Fig. 5).

As illustrated, the first housing 400 defines an inlet port 404 and an outlet port 406, with the port 404 in fluid communication with the first end 132 of the infusion line 130 and the port 406 is in fluid communication with the second end 134 of the infusion line 130. The first housing also defines a central (annular) outer passage 408 and a central (circular) inner passage 410. A first side of the diaphragm 412 is exposed to the fluid passing between the inlet port 404 and the outlet port 406, and in particular between the central outer passage 408 and the central inner passage 410.

On a second side of the diaphragm 412 is a biasing element. Unlike the embodiment of the regulator illustrated in Figs. 3-5, the biasing element is not a separate structure that is disposed in a biasing element chamber 420. Instead, the biasing element chamber 420 is filled with a gas or liquid (more particularly, a compressed gas or liquid), and defines the biasing element. In an embodiment where the housings 400, 402 are attached, then the gas or liquid may be applied directly to the second side of the diaphragm 412.

Alternatively and as illustrated in Fig. 8, a second membrane 422, for example a hydrophobic membrane, may be disposed at an end of the chamber 420 adjacent the diaphragm 412 to limit access into the chamber 420. For instance, the membrane 422 may prevent materials (e.g., fluids) from entering the chamber 420 (for example, during cleaning or a spill) in those embodiments where the first and second housings 400, 402 are separable. The membrane 422 may act on the membrane 412 in the same manner than the stopper acts on the membrane in the forgoing embodiments. As such, it will be recognized that the spacing illustrated between the membranes 412, 422 may be an exaggerated spacing so that the membranes may be distinguished from each other, and the membranes 412, 422 may well be closer, even abutting, according to other embodiments.

According to one embodiment, the gas or fluid is introduced into the biasing element chamber 420, and the chamber 420 is closed so that the gas or fluid cannot escape from the chamber. To this end, the chamber 420 may have a closed end. According to another embodiment, as is also illustrated in Fig. 8, a gas or liquid (i.e., fluid) source 426 may be mounted in the housing 150 of the infusion pump 102. For example, the source 426 may be in the form of a pump that is connected to a fluid reservoir. According to another example, the source 426 may be a valve connected to a compressed fluid reservoir, which valve can be controlled (e.g., by the controller 144) to place the chamber 420 in fluid communication with the compressed fluid reservoir. A further example may include equipment such as is illustrated in Fig. 7. Still other alternatives are possible.

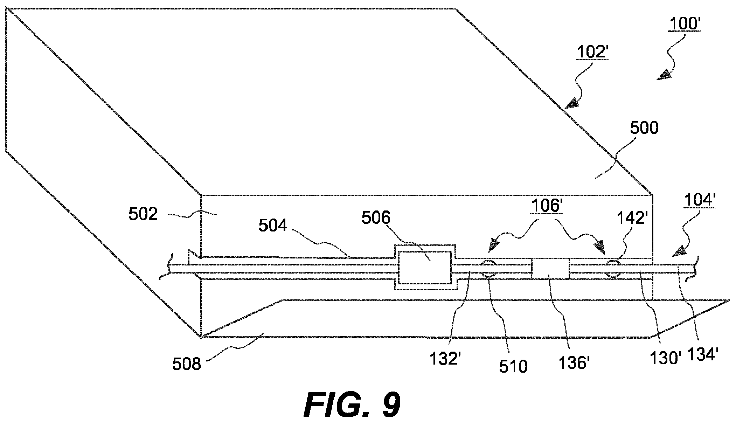

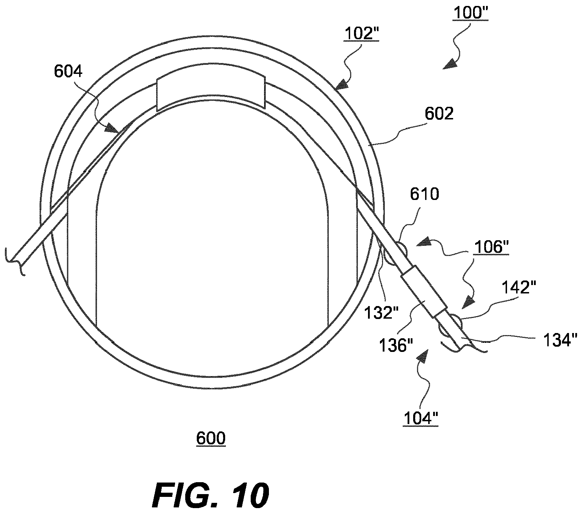

While any of the embodiments of the regulator illustrated in Figs. 3-8 may be used with the infusion pump 102 illustrated in Figs. 1 and 2 (i.e., a syringe pump), the infusion pump of the system is not limited to only syringe pumps. For example, the infusion system may include an infusion pump in the form of a volumetric pump instead. As such, embodiments of an infusion system including an infusion pump in the form of a volumetric pump are illustrated in Figs. 9 and 10. Similar structures between these embodiments and the embodiment of Figs. 1 and 2 are numbered similarly, with a prime for those structures appearing in the embodiment of Fig. 9 and a double prime for those structures appearing in the embodiment of Fig. 10.

Referring first to Fig. 9, the system 100' includes an infusion pump 102', an infusion set 104' and a sensor assembly 106'. Moreover, the infusion set 104' includes an infusion line 130' with a first end 132' and a second end 134'. A backpressure regulator 136' is disposed in the infusion line 130' between the first end 132' and the second end 134'. Moreover, the sensor assembly 106' includes a sensor 142' that is disposed downstream of the backpressure regulator 136', and that is used to determine the fluid pressure.

However, the infusion pump 102' is configured as a volumetric infusion pump having a housing 500 and a front face 502 comprising a channel, recess, or receptacle 504 in which a pump module 506 of the infusion set 104' can be received. The pump module 506 may be configured to cooperate with a wobble device that applies a wobbling action on the pump module 506 (and in particular on a membrane of the pump module) to peristaltically pump a medical fluid through the infusion set 104' towards the patient. A housing element 508 (also referred to as a door) is pivotable about a pivoting axis relative to the remainder of the housing 500, and can be moved towards the front face 502 such that in a closed state the infusion set 104' is held to the infusion pump 102'.

Tubing extends in both directions from the pump module 506. An upstream tube section connects the pump module 506 to a container (not shown) containing a medical fluid, whereas a downstream pump section connects the pump module 506 to a patient (also not shown) for delivering the medical fluid to the patient. The downstream section includes the line 130' with a backpressure regulator 136' disposed between the ends 132' and 134' of the line 130'.

The sensor assembly 106' includes a first sensor 142' that senses the pressure in a region downstream of the regulator 136'. The sensor assembly 106' also includes a sensor 510 disposed upstream of the regulator 136', which sensor 510 serves a function similar to that of the force sensor 140 in the embodiment of Figs. 1 and 2. That is, the sensor 510 can be used to determine the pressure upstream of the regulator 136', and thus be used to control the operation of the pump 102'. In fact, a sensor similar to the sensor 510 can be used in addition to or in substitution for the sensor 140 of the embodiment of Figs. 1 and 2. Consequently, this feature of the embodiment of Fig. 9 applies not only to the embodiment of Fig. 9, but to those of Figs. 1 and 2 as well.

The pump with pump module and wobble device is only one possible embodiment that may be used in an alternate embodiment of the system. It will be recognized that other volumetric infusion pump/infusion set pairs may omit the pump module 506. For example, the line 130' may instead be disposed in a channel, and a rotor or a plurality of pump fingers may act on the line 130' to force fluid in a specified direction. While one such embodiment is discussed below, other options are also possible.

Referring now to Fig. 10, the system 100" includes an infusion pump 102', an infusion set 104" and a sensor assembly 106". Moreover, the infusion set 104" includes an infusion line 130" with a first end 132" and a second end 134". A backpressure regulator 136" is disposed in the infusion line 130" between the first end 132" and the second end 134". Moreover, the sensor assembly 106" includes a sensor 142" that is disposed downstream of the backpressure regulator 136", and that is used to determine the fluid pressure.

The infusion pump 102" includes a housing 600 to which is mounted a pump head 602 having a channel, recess, or receptacle 604 in which a section of the line 130" of the infusion set 104' can be received. The pump head 602 includes a rotor with rollers that may be configured to cooperate with the section of the line 130" to peristaltically pump a medical fluid through the infusion set 104" towards the patient. The pump 102" may include additional equipment, for example, to maintain the relative position of the line 130" relative to the pump 102".

Similar to the system of Fig. 9, the system of Fig. 10 includes a sensor 610 disposed upstream of the backpressure regulator 136". It is believed that the sensor 610 can be used like the sensor 140 in the embodiment of Figs. 1 and 2, or the sensor 510 of the embodiment of Fig. 9. As such, the sensor assembly 106" of the embodiment of Fig. 10 may include both the sensor 142" and the sensor 610.

Thus, an improved infusion system and method has been described. The description provided above is intended for illustrative purposes, and is not intended to limit the scope of the disclosure to any particular method, system, apparatus, or device described herein. For example, still further embodiments may include pneumatically-driven volumetric pumps as part of the infusion system and method.

### Aspects

Aspect 1. An infusion system comprising:
an infusion device,
an infusion set comprising an infusion line with a first end and a second end, and
a backpressure regulator disposed between the first end and the second end of the infusion line, the infusion device configured to pump a medical fluid through the infusion line, and
a sensor assembly comprising a first sensor disposed along the infusion line between the backpressure regulator and the second end of the infusion line, wherein the infusion device comprises a controller coupled to the sensor assembly.

Aspect 2. The infusion system according to Aspect 1, wherein the controller is configured to receive a signal from the first sensor, to determine if an occlusion has occurred downstream of the regulator based on the signal, and to control the infusion device to pause delivery of fluid if an occlusion has occurred.

Aspect 3. The infusion system according to Aspect 1 or 2, wherein backpressure regulator comprises a diaphragm and a biasing element that biases the diaphragm, wherein the biasing element is adjustable.

Aspect 4. The infusion system according to Aspect 3, wherein the biasing element is manually adjustable.

Aspect 5. The infusion system according to Aspect 3, wherein the biasing element is automatically adjustable.

Aspect 6. The infusion system according to any one of Aspects 1 to 5, wherein backpressure regulator comprises a diaphragm and a biasing element that biases the diaphragm, wherein the biasing element is a spring.

Aspect 7. The infusion system according to any one of Aspects 1 to 5, wherein the backpressure regulator comprises a diaphragm and a biasing element that biases the diaphragm, wherein the biasing element comprises a volume of compressed fluid.

Aspect 8. The infusion system according to Aspect 7, wherein the volume is in fluid communication with a pump.

Aspect 9. The infusion system according to Aspect 8, further comprising a sensor to determine the pressure of the fluid in the volume, the sensor coupled to the controller and the controller configured to operate the pump to vary the pressure of the fluid in the volume.

Aspect 10. The infusion system according to any one of Aspects 1 to 9, wherein the sensor assembly comprises a second sensor disposed along the infusion line between the first end of the infusion line and the backpressure regulator.

Aspect 11. The infusion system according to any one of Aspects 1 to 10, wherein:
the infusion device comprises a syringe including a barrel with an outlet end and a plunger, and a pusher device in engagement with the plunger, and the first end of the infusion line is in communication with the outlet end of the barrel, and
the sensor assembly comprises a second sensor disposed between the plunger and the pusher device.

Aspect 12. The infusion system according to Aspect 11, wherein the plunger comprises a first end disposed outside the barrel, and a second end with a piston disposed within the barrel, the second sensor disposed between the first end of the plunger and the pusher device.

Aspect 13. The infusion system according to Aspect 11 or 12, wherein the controller is configured to receive a signal from the second sensor, and to control the pusher device to deliver fluid from the syringe based on the signal.

Aspect 14. The infusion system according to any one of Aspects 1 to 10, wherein the infusion device comprises a volumetric pump.

Aspect 15. The infusion system according to any one of Aspects 1 to 14, wherein the first sensor is disposed outside the infusion line.

Aspect 16. The infusion system according to Aspect 15, wherein the infusion device comprises a housing, and the first sensor is attached to the housing.

Aspect 17. The infusion system according to any one of Aspects 1 to 16, wherein the backpressure regulator is attached at least in part to the housing.

Aspect 18. The infusion system according to Aspect 17, wherein the backpressure regulator comprises a first housing and a diaphragm joined to the first housing, the first housing being part of the infusion line, and a second housing and a biasing element in the second housing, the second housing mounted on the infusion device.

Aspect 19. The infusion system according to any one of Aspects 1 to 18, wherein the first sensor is a force sensor.

Aspect 20. The infusion system according to any one of Aspects 1 to 18, wherein the first sensor is a pressure sensor.

## Claims

1. An infusion system comprising:
an infusion device,
an infusion set comprising an infusion line with a first end and a second end, and
a backpressure regulator disposed between the first end and the second end of the infusion line, the infusion device configured to pump a medical fluid through the infusion line, and
a sensor assembly comprising a first sensor disposed along the infusion line between the backpressure regulator and the second end of the infusion line,
wherein the infusion device comprises a controller coupled to the sensor assembly.

2. The infusion system according to claim 1, wherein the controller is configured to receive a signal from the first sensor, to determine if an occlusion has occurred downstream of the regulator based on the signal, and to control the infusion device to pause delivery of fluid if an occlusion has occurred.

3. The infusion system according to claim 1 or 2, wherein the backpressure regulator comprises a diaphragm and a biasing element that biases the diaphragm, wherein the biasing element is manually or automatically adjustable.

4. The infusion system according to any one of claims 1 to 3, wherein the backpressure regulator comprises a diaphragm and a biasing element that biases the diaphragm, wherein the biasing element is a spring.

5. The infusion system according to any one of claims 1 to 3, wherein the backpressure regulator comprises a diaphragm and a biasing element that biases the diaphragm, wherein the biasing element comprises a volume of compressed fluid.

6. The infusion system according to claim 5, wherein the volume is in fluid communication with a pump, and further comprising a sensor to determine the pressure of the fluid in the volume, the sensor coupled to the controller and the controller configured to operate the pump to vary the pressure of the fluid in the volume.

7. The infusion system according to any one of claims 1 to 6, wherein the sensor assembly comprises a second sensor disposed along the infusion line between the first end of the infusion line and the backpressure regulator.

8. The infusion system according to any one of claims 1 to 6, wherein:
the infusion device comprises a syringe including a barrel with an outlet end and a plunger, and a pusher device in engagement with the plunger, and the first end of the infusion line is in communication with the outlet end of the barrel, and
the sensor assembly comprises a second sensor disposed between the plunger and the pusher device.

9. The infusion system according to claim 8, wherein the plunger comprises a first end disposed outside the barrel, and a second end with a piston disposed within the barrel, the second sensor disposed between the first end of the plunger and the pusher device.

10. The infusion system according to claim 8 or 9, wherein the controller is configured to receive a signal from the second sensor, and to control the pusher device to deliver fluid from the syringe based on the signal.

11. The infusion system according to any one of claims 1 to 7, wherein the infusion device comprises a volumetric pump.

12. The infusion system according to any one of claims 1 to 11, wherein the first sensor is disposed outside the infusion line.

13. The infusion system according to claim 12, wherein the infusion device comprises a housing, and the first sensor is attached to the housing.

14. The infusion system according to claim 13, wherein the backpressure regulator is attached at least in part to the housing, and the backpressure regulator comprises a first housing and a diaphragm joined to the first housing, the first housing being part of the infusion line, and a second housing and a biasing element in the second housing, the second housing mounted on the infusion device.

15. The infusion system according to any one of claims 1 to 14, wherein the first sensor is a force sensor or a pressure sensor.
